# EUROPEAN PATENT SPECIFICATION

(11) **EP 3 067 023 B1**
(45) Date of publication and mention of the grant of the patent: **27.06.2018**
(21) Application number: 16159013.8
(22) Date of filing: 07.03.2016
(51) Int. Cl.: A61F 13/00, A61F 5/01, A61F 13/02

(54) **SYSTEM, METHOD, AND DEVICE FOR SUPPORTING A BODY PART**
SYSTEM, VERFAHREN UND VORRICHTUNG ZUM ABSTÜTZEN EINES KÖRPERTEILES
SYSTÈME, PROCÉDÉ ET DISPOSITIF POUR SUPPORTER UNE PARTIE DU CORPS

(30) Priority: 10.03.2015 US 201562130816 P
(43) Date of publication of application: 14.09.2016
(73) Proprietor: Spidertech Inc., Toronto, Ontario M1P 4W9 (CA)
(72) Inventor: ARBESMAN, Ray, Toronto, Ontario M5P 1V2 (CA); MACKELVIE, Winston, Knowlton, Québec J0E 1V0 (CA)
(74) Representative: Goodman, Simon John Nye

(56) References cited:
- EP-A2- 0 051 935
- WO-A1-2014/190416
- US-A1- 2011 015 556
- US-B2- 8 742 196

## Description

### CROSS-REFERENCES TO RELATED APPLICATIONS

This application claims priority from United States provisional patent application no. 62/130,816, filed on March 10, 2015.

### FIELD

The disclosure relates to systems, methods, and articles for supporting body parts, such as the lower back. More specifically, the disclosure relates to precut kinesiology tapes and systems including precut kinesiology tapes.

### BACKGROUND

U.S. Patent no. 8,742,196 (Arbesman et al.) purports to disclose an adhesive support article for supporting a body part of a user. A single-sided stretchable adhesive tape blank is precut into a sheet having a central anchoring portion and a plurality of outwardly extending fingers. The sheet is marked with visual indicators to guide the user of the support article for applying the central anchoring portion onto a first exterior surface of the body part and for stretching the fingers before adhering them at a predetermined distance away from the central anchoring portion. The adhesive support article can also be provided in a support system with at least one corresponding body landmark article, providing further guidance for applying the support article to the desired body part. WO 2014/190416 A1 describes a user-configurable precut kinesiology tape strip provided for application by a user to a patient. Each end of the strip is lobed, having troughs between the lobes. The lobes are in pairs opposite each other.

EP 0 051 935 A2 describes a relatively thin polymeric film which is conformable to animal anatomical surfaces. A pressure sensitive adhesive is attached to at least a portion of one surface of said film, and a release liner is attached to the adhesive coated surface of said film. The film also comprises a releasable layer attached to the surface of said film opposite the surface containing said pressure sensitive adhesive, said releasable layer being attached to said film more tenaciously than the release liner is attached to the adhesive surface of said film.

U.S. patent application publication no. 2011/0015556 (Fabo et al.) purports to disclose a stiffening layer for facilitating application of a plastic film to skin. The plastic film is a component in a wound dressing or other medical device and has the stiffening layer removably attached to one side thereof. At least a part of the other side of the film is provided with a layer of self-adhering adhesive. The stiffening layer is made of a stretchable material having a thickness of between 0.5-10 mm. The stiffening layer covers the whole area of the film and is divided into two or more portions by cutting line or lines.

### SUMMARY

The invention is defined in the appended independent claims, to which reference should now be made. Preferred or advantageous features of the invention are set out in the dependent sub-claims.

The following summary is intended to introduce the reader to various aspects of the teachings of this document, but not to delimit any invention.

Embodiments of the invention may advantageously be used in a method for supporting a body part. The method comprises a) removing a first release liner portion from an adhesive side of an anchor portion of a precut piece of kinesiology tape; b) adhering the adhesive side of the anchor portion to a generally flat surface of the body part; c) removing a second release liner portion from an adhesive side of a finger portion of the precut piece of kinesiology tape, while retaining a finger reinforcement layer on a non-adhesive side of the finger portion; d) after step c), adhering the adhesive side of the finger portion to the generally flat surface, so that the precut piece of kinesiology tape lies generally flat on the generally flat surface; and e) after step d), removing the finger reinforcement layer from the non-adhesive side of the finger portion.

In some examples, the body part may be a back, and more specifically may be a lower back.

In some examples, step b) may comprise adhering the adhesive side of the anchor portion so that the anchor portion extends laterally across the lower back. Step d) may comprise adhering the adhesive side of the finger portion so that the finger portion extends upwardly along the lower back from the anchor portion.

In some examples, step d) may comprise using the finger reinforcement layer to inhibit stretching of the finger portion while adhering the adhesive side of the finger portion to the generally flat surface.

In some examples, the anchor portion may comprise an anchor reinforcement layer on a non-adhesive side of the anchor portion. Step a) may further comprise retaining the anchor reinforcement layer on the non-adhesive side of the anchor portion. The method may further comprise removing the anchor reinforcement layer from the non-adhesive side of the anchor portion after step b).

In some examples, the method may further comprise i) removing an additional release liner portion from an adhesive side of an additional finger portion of the precut piece of kinesiology tape, while retaining an additional finger reinforcement layer on a non-adhesive side of the additional finger portion; ii) after step i), adhering the adhesive side of the additional finger portion to the generally flat surface, so that the precut piece of kinesiology tape lies generally flat on the generally flat surface; and iii) after step ii), removing the additional finger reinforcement layer from the non-adhesive side of the additional finger portion.

In some examples, one or more of the finger reinforcement layer and the additional finger reinforcement layer may comprise instructions printed thereon. The method may comprise adhering the finger portion and the additional finger portion in an order that accords with the instructions.

Embodiments of the invention may also advantageously be used in another method for supporting a body part. The method comprises a) removing a first release liner portion from an adhesive side of an anchor portion of a precut piece of kinesiology tape; b) adhering the adhesive side of the anchor portion to a surface of the body part; c) removing a second release liner portion from an adhesive side of a finger portion of the precut piece of kinesiology tape; d) using a finger reinforcement layer on a non-adhesive side of the finger portion to inhibit stretching of the finger portion while adhering the adhesive side of the finger portion to the surface of the body part; and e) after step d), removing the finger reinforcement layer from the non-adhesive side of the finger portion.

In some examples, step b) may comprise using an anchor reinforcement layer on a non-adhesive side of the anchor portion to inhibit stretching of the anchor portion while adhering the adhesive side of the anchor portion to the surface of the body part. The method may further comprise removing the anchor reinforcement layer from the non-adhesive side of the anchor portion.

In some examples, the surface of the body part may be generally flat. For example, the body part may be a back, or more specifically may be a lower back.

In some examples, step b) may comprise adhering the adhesive side of the anchor portion so that the anchor portion extends laterally across the lower back. Step d) may comprise adhering the adhesive side of the finger portion so that the finger portion extends upwardly along the lower back from the anchor portion.

In some examples, the method may further comprise i) removing an additional release liner portion from an adhesive side of an additional finger portion of the precut piece of kinesiology tape; ii) using an additional finger reinforcement layer on a non-adhesive side of the additional finger portion to inhibit stretching of the additional finger portion while adhering the adhesive side of the additional finger portion to the surface of the body part; and iii) after step ii), removing the additional finger reinforcement layer from the non-adhesive side of the finger portion.

In some examples, one or more of the finger reinforcement layer and the additional finger reinforcement layer may comprise instructions printed thereon. The method may further comprise adhering the finger portion and the additional finger portion in an order that accords with the instructions.

According to another aspect, a support article for the lower back is disclosed. The support article comprises a precut piece of kinesiology tape having an adhesive side and a non-adhesive side. The precut piece of kinesiology tape comprises an anchor portion for adhering to the lower back, and a plurality of finger portions extending transversely from a first side of the anchor portion for adhering to the lower back. A release liner is on the adhesive side of the precut piece of kinesiology tape. The release liner is weakened along one or more junctions between the anchor portion and the finger portions. A stretch-inhibiting reinforcement layer is adhered to the non-adhesive side of the precut piece of kinesiology tape. The stretch-inhibiting reinforcement layer is weakened along the one or more junctions between the anchor portion and the finger portions or is provided as separate pieces on the anchor portion and the finger portions.

In some examples, the finger portions may extend from only the first side of the anchor portion.

In some examples, the release liner has a release liner thickness, and the stretch-inhibiting reinforcement layer has a reinforcement layer thickness less than the release liner thickness.

In some examples, the support article may further comprise a first set of instructions printed on the stretch inhibiting reinforcement layer. The support article may further comprise a second set of instructions printed on the release liner.

In some examples, the stretch-inhibiting reinforcement layer is a paper layer.

In some examples, the release liner is perforated along the one or more junctions.

In some examples, the release liner includes fold lines thereon for facilitating folding of the release liner during removal.

According to another aspect, a system for supporting the lower back is disclosed. The system comprises a precut piece of kinesiology tape having an adhesive side and a non-adhesive side. The precut piece of kinesiology tape comprises an anchor portion for adhering to the lower back, and a plurality of finger portions extending transversely from a first side of the anchor portion for adhering to the lower back. A release liner is on the adhesive side of the precut piece of kinesiology tape. The release liner is weakened along one or more junctions between the anchor portion and the finger portions. A stretch-inhibiting reinforcement layer is adhered to the non-adhesive side of the precut piece of kinesiology tape. The stretch-inhibiting reinforcement layer comprises a plurality of finger reinforcement layers and an anchor reinforcement layer. The system further comprises instructions directing a user to remove each finger reinforcement layer from each respective finger portion after the respective finger portion has been adhered to the lower back.

### BRIEF DESCRIPTION OF THE DRAWINGS

The drawings included herewith are for illustrating various examples of articles, methods, and apparatuses of the present specification and are not intended to limit the scope of what is taught in any way. In the drawings:
Figure 1 is a rear perspective view of an example support article;
Figure 2 is a front perspective view of the support article of Figure 1;
Figure 3 is a rear plan view of the support article of Figure 1;
Figure 4 is a side view of the support article of Figure 1, with the encircled region shown in enlarged form;
Figure 5 is a front plan view of the support article of Figure 1;
Figure 6 is a rear perspective view of the support article of Figure 1, showing the release liner partially removed from the tape;
Figure 7 is a front perspective view of the support article of Figure 1, showing the reinforcement layer partially removed from the tape;
Figure 8 is a front plan view of the tape of the support article of Figure 1;
Figures 9 to 16 show a series of steps for applying the support article of Figure 1 to a patient's back;
Figure 17 is a rear perspective view of an alternative example support article, having a different shape than the support article of Figure 1;
Figure 18 is a front plan view of the support article of Figure 17;
Figure 19 is a front plan view of the tape of the support article of Figure 17;
Figure 20 is a front plan view of an alternative example support article, wherein the reinforcement layer is weakened along a junction between the anchor portion and finger portions of the tape
Figure 21 is a front perspective view of an alternative example support article, wherein the reinforcement layer is provided as separate pieces; and
Figure 22 is a front perspective view of a finger reinforcement layer of the support article of Figure 21.

### DETAILED DESCRIPTION

Various apparatuses or processes will be described below to provide an example of an embodiment of the claimed subject matter. No embodiment described below limits any claim and any claim may cover processes or apparatuses that differ from those described below. The claims are not limited to apparatuses or processes having all of the features of any one apparatus or process described below or to features common to multiple or all of the apparatuses described below. It is possible that an apparatus or process described below is not an embodiment of any exclusive right granted by issuance of this patent application. Any subject matter described below and for which an exclusive right is not granted by issuance of this patent application may be the subject matter of another protective instrument, for example, a continuing patent application, and the applicants, inventors or owners do not intend to abandon, disclaim or dedicate to the public any such subject matter by its disclosure in this document.

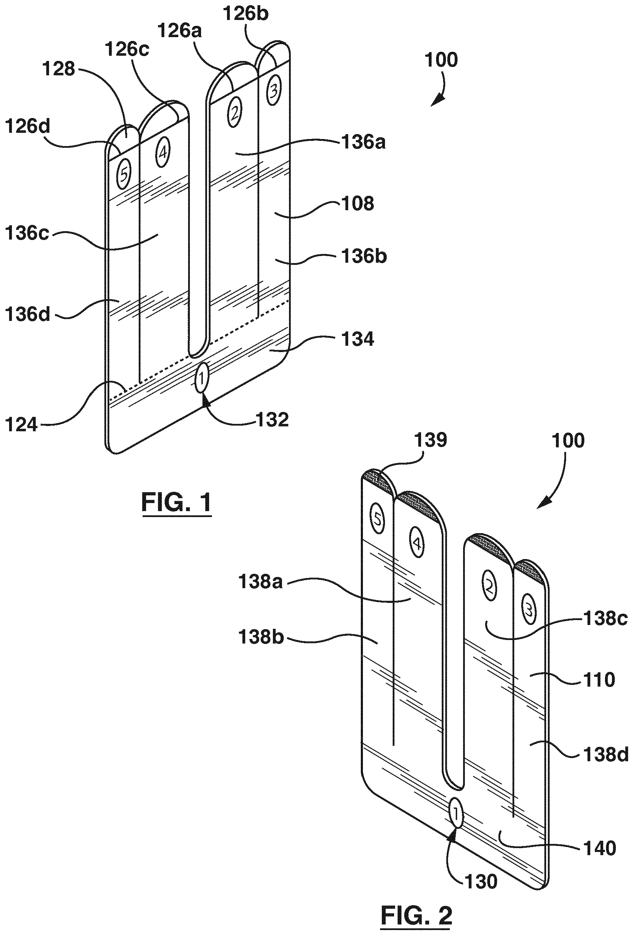

Referring to Figures 1 to 8, an example support article 100 is shown. As will be described in further detail below, the support article 100 may be used, for example, to support a body part, such as but not limited to the lower back.

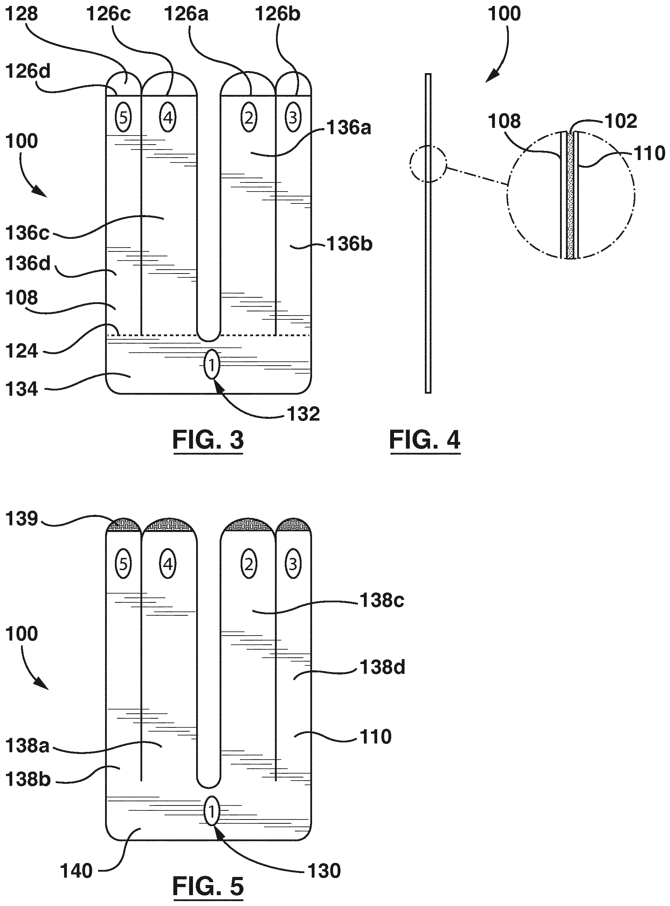

In the example shown, the support article 100 includes a precut piece of kinesiology tape 102 (also referred to as tape 102), shown in Figures 4, and 6 to 8. The tape 102 may be any suitable kinesiology tape, for example one including a high elasticity woven strip (i.e. having a stretch ratio of about 1.4 along its length) having an adhesive on one side thereof. In some examples, the strip may be made from cotton and the adhesive may be an acrylic adhesive suitable for use on skin.

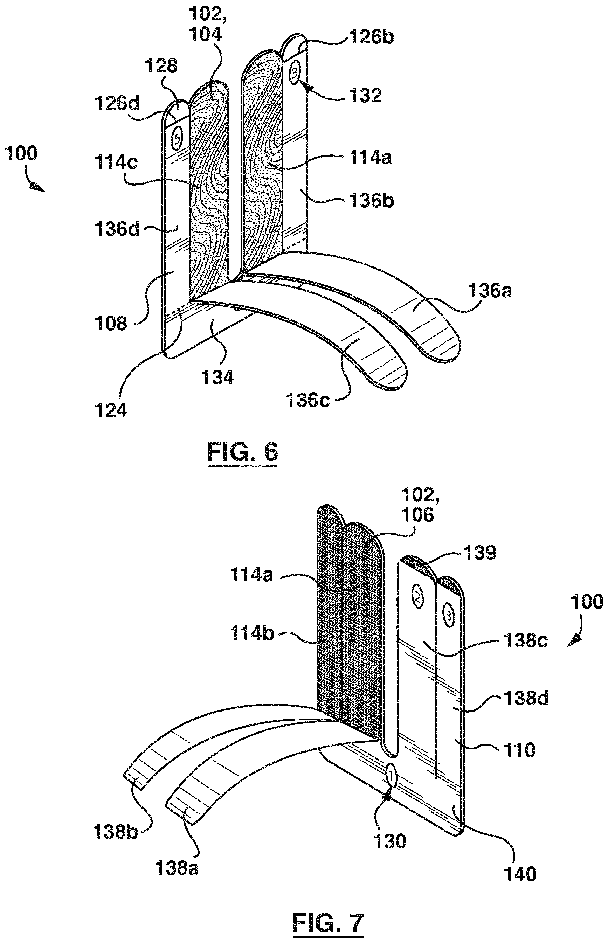

The side of the tape 102 having the adhesive may be referred to as an adhesive side 104 (shown in Figure 6), and the opposite side of the tape may be referred to as a non-adhesive side 106 (Shown in figure 7).

Referring to Figures 1, 3, and 6, in the example shown, the support article 100 further includes a release liner 108 on the adhesive side 104 of the tape 102. The release liner 108 protects the adhesive side during storage, and may be removed in order to apply the tape 102 to a body part.

Referring to Figures 2, 5, and 7, in the example shown, the support article 100 further includes a reinforcement layer 110 on the non-adhesive side 106 of the tape 102. The reinforcement layer 110 is removably adhered to the non-adhesive side 106, and when removed from the non-adhesive side 106, generally does not leave adhesive on the non-adhesive side 106. As will be described in further detail below, the reinforcement layer 110 may assist with application of the tape 102 to a body part, for example by stiffening the tape 102, inhibiting folding of the tape 102 onto itself and sticking to itself, and/or inhibiting stretching of the tape 102 during application. In some particular examples, in which the reinforcement layer 110 inhibits stretching of the tape 102, the reinforcement layer 110 may also be referred to as a "stretch-inhibiting reinforcement layer".

In some particular examples, the reinforcement layer 110 may be a paper layer. In such examples, as paper is generally not stretchable, the reinforcement layer may inhibit stretching of the tape 102. The paper layer may have a thickness (also referred to as a reinforcement layer thickness) of, for example, about 0.1 mm. Furthermore, in some examples, the reinforcement layer thickness may be less than the thickness of the release liner (also referred to as the release liner thickness). For example, the reinforcement layer thickness may be between about 0.05 mm and about 0.15 mm, and the release liner thickness may be between about 0.18 mm and about 0.28 mm.

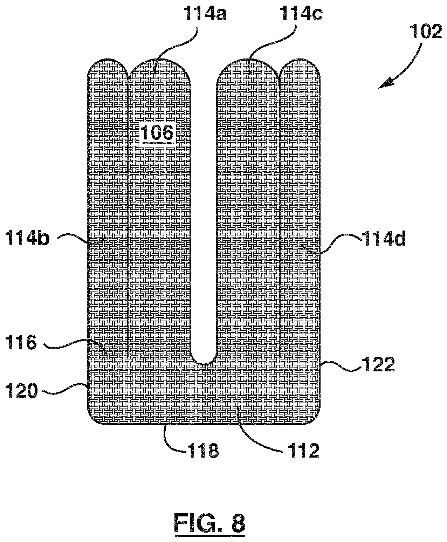

As noted above, in the example shown the tape 102 is precut. Specifically, referring to Figure 8, the tape 102 is precut to include an anchor portion 112, and a plurality of finger portions 114a-114d extending from the anchor portion 112. The anchor portion 112 is generally rectangular, and includes first 116 and second 118 opposed sides, and first 120 and second 122 opposed ends. In the example shown in Figure 8, the finger portions 114a-114d extend transversely from only the first side 116 of the anchor portion 112. As will be described in detail below, in use, the anchor portion 112 may be adhered to the lower back so that the anchor portion 112 extends laterally across the lower back, and the finger portions 114a-114d may be adhered to the lower back above the anchor portion 112 so that the finger portions 114a-114d extend upwardly along the lower back from the anchor portion 112.

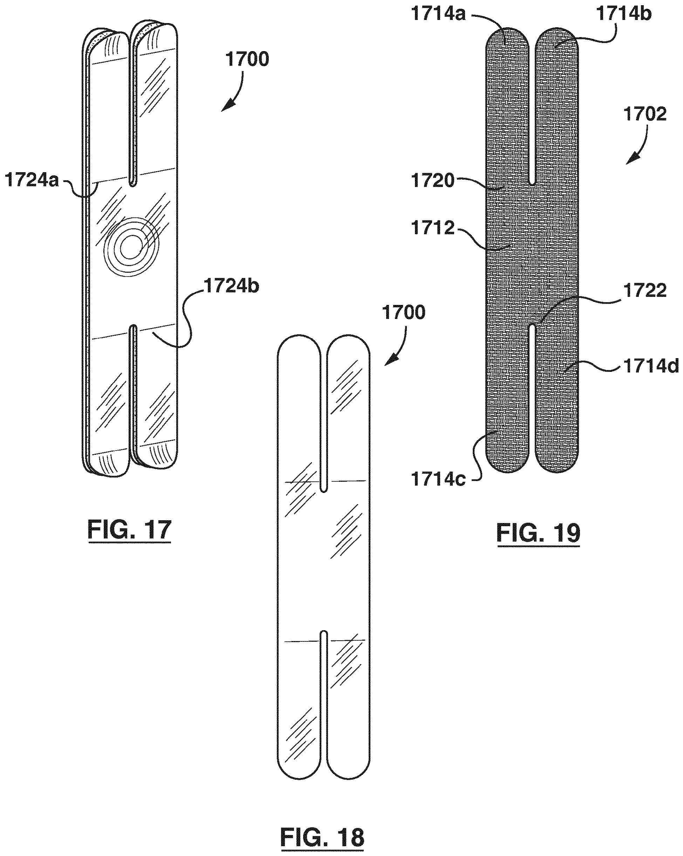

In alternative examples, the tape may be precut into another shape, For example, referring to Figures 17 to 19, an alternative support article 1700 is shown. In Figures 17 to 19, like reference numerals, incremented by 1600, are used to refer to like features as in Figures 1 to 8. In the support article 1700, the tape 1702 includes two finger portions 1714a, 1714b, extending from the first 1720 end of the anchor portion 1712, and two finger portions 1714c, 1714d extending from the second end 1722 of the anchor portion 1712.

In further alternative examples, the tape may be of other shapes. For example, the finger portions may extend from both sides of the anchor portion, and/or only one end of the anchor portion. For further example, the anchor portion may be another shape, such as square.

Referring back to Figures 1, 3, and 6, the release liner 108 may be weakened along one or more junctions between the anchor portion 112 and the finger portions 114a-114d. In the example shown in Figures 1 to 8, the release liner is weakened along a single line 124 extending along a junction between the anchor portion 112 and the finger portions 114a-114d. In the example shown in Figures 17 to 19, the release liner is weakened along two lines 1724a, 1724b, each extending along one junction between the anchor portion 1712 and the finger portions 1714.

The release liner 108 may be weakened, for example, by perforating or scoring the release liner 108 along the junction.

Weakening the release liner 108 along the junction between the anchor portion 112 and the finger portions 114a-114d may facilitate stepwise application of the tape 102 to the body. For example, as will be described in further detail below, the release liner 108 may be severed along the line 124, to separate the portion 134 of the release liner 108 that is on the anchor portion 112 (also referred to herein as the first release liner portion 134) from the portions 136a-136d of the release liner 108 that are on the finger portions 114a-114d (also referred to as second release liner portions 136a-136d). The portion 134 of the release liner 108 that is on the anchor portion 112 may then be removed, and the anchor portion 112 may be applied to the body. The portions 136a-136d of the release liner 108 that are on the finger portions 114 may then be removed, and the finger portions 114a-114d may then be applied to the body.

Referring back to Figures 2, 5, and 7, in the example shown, the reinforcement layer 110 covers substantially all of the non-adhesive side 106 of the tape 102 (i.e. covers both the finger portions 114a-114d and the anchor portion 112). Specifically, the reinforcement layer 110 includes finger reinforcement layers 138a-138d on the respective finger portions 114a-114d, and an anchor reinforcement layer 140 on the anchor portion 112. The finger reinforcement layers 138a-138d cover the finger portions 114a-114d, but for a small uncovered portion 139 at the tip of the finger portions 114a-114d. The portion 139 is left uncovered to facilitate gripping of the finger reinforcement layers 138a-138d, for removal of the finger reinforcement layers 138a-138d from the tape 102.

In alternative examples, the reinforcement layer 110 may cover only a portion of the non-adhesive side 106 of the tape 102. For example, the reinforcement layer 110 may be only on the finger portions 114 (i.e. may include only the finger reinforcement layers 138a-138d).

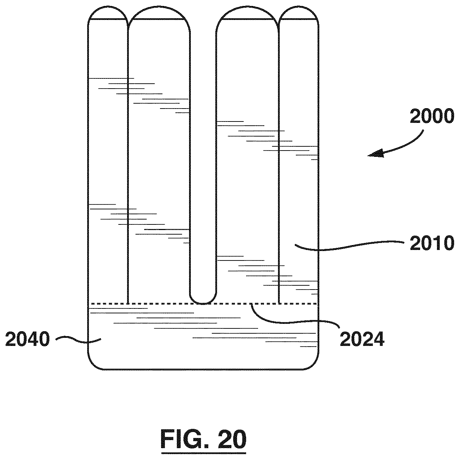

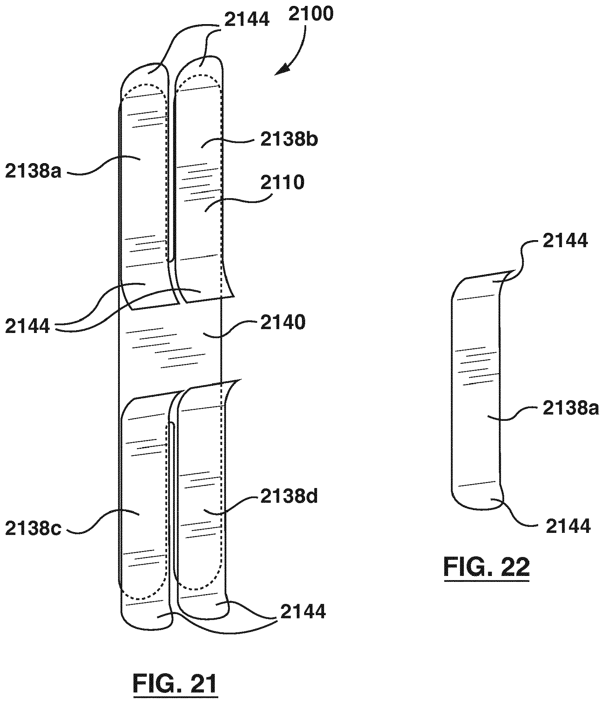

In the support article 100 shown in Figures 1 to 8, the reinforcement layer 110 is provided as a single piece without any weakened portions. An alternative support article 2000 is shown in Figure 20, wherein like features to Figures 1 to 8 are labelled with like reference numbers, incremented by 1900. In the support article 2000, the reinforcement layer 2010 is provided as a single piece and is weakened along line 2024 along a junction between the anchor portion and the finger portions, to facilitate stepwise removal of the reinforcement layer 2010. A further alternative support article is 2100 is shown in Figure 21, wherein like features to Figures 1 to 8 are labelled with like reference numbers, incremented by 2000. In the support article 2100, the reinforcement layer 2110 is provided as separate pieces, including an anchor reinforcement layer 2140 on the anchor portion and finger reinforcement layers 2138a to 2138d on the finger portions. A finger reinforcement layer 2138a, removed from the tape, is shown in Figure 22. In this example, the finger reinforcement layers 2138a to 2138d include tabs 2144 which extend beyond the finger portions 2114a to 2114b. The tabs in some examples do not include any adhesive. In alternative examples, the tabs may include adhesive, for example for ease of manufacturing. The tabs 2144 are provided at opposed ends of each finger reinforcement layer 2138a to 2138b. The tabs 2144 may be gripped when applying the finger portion to the patient, so that the finger portions 2114a to 2114b themselves do not necessarily need to be gripped, and so as to prevent or reduce the risk of soiling of the adhesive on the finger portions 2114a to 2114b. The tabs 2144 may also be gripped in order to remove the finger reinforcement layers 2138a to 2138d from the tape.

Referring back to Figures 1 and 3, in the example shown, the release liner 108 includes fold lines 126a-126d thereon, to facilitate folding of the release liner 108 during removal. The fold lines 126a-126d may be, for example, printed on the release liner 108 to indicate to a user where the release liner 108 may be folded. Alternatively or in addition, the fold lines 126a-126d may include a crease that is formed in the release liner 108. In use, as will be described in further detail below, each second release liner portion 136a-136d may be removed from the respective finger portions 114a-114d by peeling the second release liner portions 136a-136d upwardly from the line 124 towards the respective fold lines 126a-126d. When the second release liner portions 136a-136d have been peeled to the respective fold lines 126a-126d, peeling may be stopped, and the release liner 108 may be folded at the fold lines 126a-126d, either by creating a crease in the release liner 108 along fold lines 126a-126d, or by folding the release liner 108 along a crease already in the release liner 108 at fold lines 126a-126d. The portions of the release liner above the fold lines 126a-126d, which will still be adhered to the tape 102, may provide a gripping portion 128 (only one gripping portion is labelled in the figures) which may be gripped by the user without having the tape 102 adhere to their fingers. After the remainder of each finger portion 114a-114d (i.e. the portion below the fold lines 126a-126d) has been applied to a patient, the gripping portion 128 may be removed from the finger portions 114a-114d.

Referring to Figures 1 and 2, in the example shown, a first set of instructions 130 is printed on the reinforcement layer 110 (shown in Figure 2), and a second set of instructions 132 is printed on the release liner 108 (shown in Figure 1). The instructions may guide a user in applying the tape 102 to the body in a stepwise fashion.

In the example shown, the instructions include the numeral 1 printed both on the portion of the release liner 108 covering the anchor portion 112 (i.e. the first release liner portion 134), and on the portion of the reinforcement layer 110 on the anchor portion 112 (i.e. the anchor reinforcement layer 140). The instructions further include the numerals 2 to 5, respectively, printed both on the portions of the release liner covering the finger portions 114a-114d (i.e. the second release liner portions 136a-d, respectively), and on the portions of the reinforcement layer 110 on the finger portions 114a-114d (i.e. the finger reinforcement layers 138a-138d, respectively). As will be described in further detail below, in use, the instructions 130, 132 guide the user in first applying the anchor portion 112 to the body, and then sequentially applying each of the finger portions 114 to the body.

In the example shown, as both the reinforcement liner 110 and release liner 108 include instructions (130, 132 respectively), the user may see the instructions both when viewing the support article 100 from the rear and when viewing the support article 100 from the front. Accordingly, the user may view the instructions both when removing the release liner 108, and when applying the tape 102 to the body (i.e. when the release liner is facing away from the user and the reinforcement layer 110 is visible). This may allow for ease of application.

In alternative examples, the instructions could be on only one of the release liner 108 and the reinforcement layer 110. In further alternative examples, the instructions could be provided separately from the release liner 108 and reinforcement layer 110. For example, the instructions could be separate from the support article 100 and packaged and sold with the support article 100.

As will be described below, it may in some instances be desirable to retain the finger reinforcement layers 138a-138d on the tape during application of the tape 102. As such, in some examples, instructions (not shown) may be provided that direct the user to remove each finger reinforcement layer 138a-138d from each respective finger portion 114a-114d after the respective finger portion 114a-114d has been adhered to the body. These instructions may be printed on the support article (e.g. on the release liner 108 and/or the reinforcement layer 110), or provided separately (e.g. packaged with the support article).

The support article 100 and any instructions (whether printed on the support article 100 or separate from the support article 100) may be referred to collectively as a system for supporting a body part.

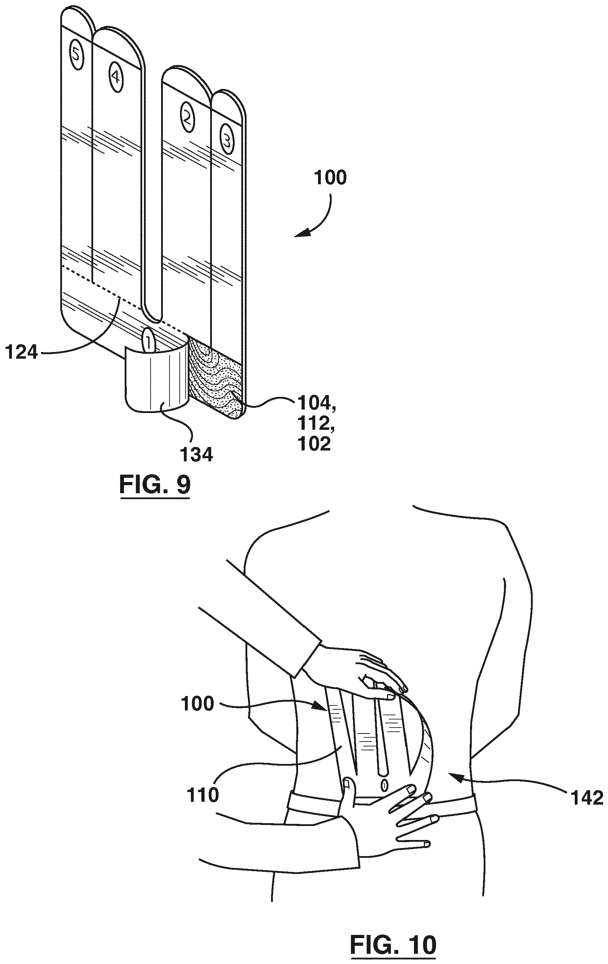

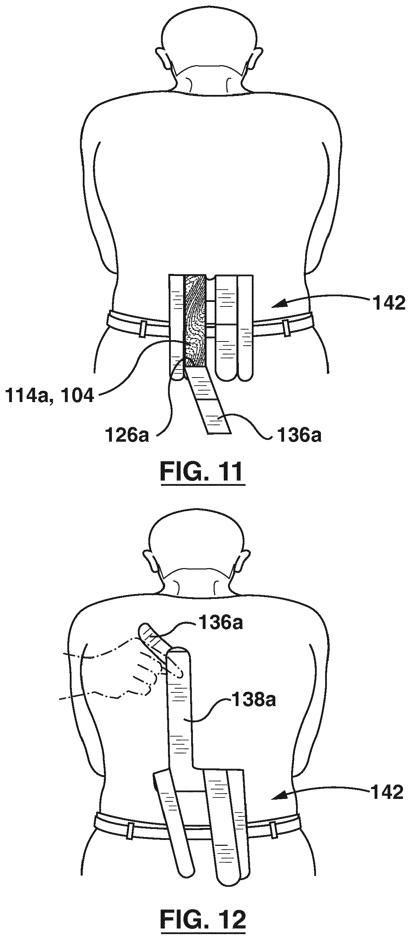

Referring now to Figures 9 to 16, an example method for supporting a body part with support article 100 will be described. For simplicity, the first 130 and second 132 set of instructions are not shown in Figures 11 to 16.

It has been determined that the support article 100 may be particularly useful in supporting body parts having generally flat surfaces, as the reinforcement layer 110 may hold the finger portions 114 of the tape 102 in a generally flat state during application. As used herein, the phrase "body parts having generally flat surfaces" refers to body parts to which the tape 102 may be applied without wrapping the tape 102 around the body part, either substantively or entirely. For the purposes of support article 100, body parts with generally flat surfaces include the back, and more particularly the lower back, the thigh, the abdomen, and the chest. In alternative examples, however, the support article 100 may be used on body parts with non-flat surfaces.

In the example shown in Figures 9 to 16, the support article 100 is used to support the lower back 142 of a patient. Referring to Figure 9, as a first step, the first release liner portion 134 may be removed from the adhesive side 104 of the anchor portion 112 of the tape 102, for example by tearing the release liner along line 124. Referring to Figure 10, the adhesive side 104 of the anchor portion 112 may then be adhered to the lower back 142, so that it extends laterally across the lower back 142.

Referring to Figures 11 and 12, the second release liner portion 136a may then be removed from the adhesive side 104 of the finger portion 114a (i.e. the finger portion 114a labelled with the numeral 2), and the adhesive side 104 of the finger portion 114a may be adhered to the lower back 142, so that it lies generally flat on the lower back 142, and extends upwardly along the lower back 142 from the anchor portion 112. When the second release liner portion 136a is removed from the adhesive side 104 of the finger portion 114a, it may optionally be removed only partially at first, for example by peeling the second release liner portion 136a to the fold line 126a and then creasing the release liner portion 136a at the fold line. As described above, this may create a gripping portion 128 which may be gripped by the user without having the tape 102 adhere to their fingers.

During removal of the second release liner portion 136a and application of the finger portion 114a, the finger reinforcement layer 138a is retained on the non-adhesive side 106 of the finger portion 114a, and is used to inhibit stretching of the finger portion 114a while adhering the adhesive side 104 of the finger portion 114a to the lower back 142. More specifically, it has been determined that when supporting a body part with a kinesiology tape, it can be beneficial to apply the tape without stretching the tape. However, due to the high-stretch nature of the tape, it can be difficult to apply the tape without stretching the tape. By retaining the finger reinforcement layer 138a on the finger portion 114a during application, stretching of the tape 102 is inhibited, thereby facilitating proper application of the tape 102.

It will be appreciated that although the reinforcement layer generally inhibits stretching, the tape may still stretch to a small degree, and/or stretch at particular locations. For example, in order to apply the finger portions 114a-114b at a particular angle with respect to the anchor portion 112, the finger portions 114a-114b may be bent near the junction of the finger portions 114a-114b and the anchor portion 112. In order to bend the finger portions 114a-114b, some stretch will be applied to the finger portions 114a-114b in the area of the bend.

Furthermore, by retaining the finger reinforcement layer 138a on the finger portion 114a during application, folding of the finger portion 114a onto itself and sticking to itself may be inhibited.

After the majority of each finger portion 114a-114d (i.e. the portion not covered by the gripping portion 128) has been applied to a patient, the gripping portion 128 may be removed from the finger portions 114a-114d.

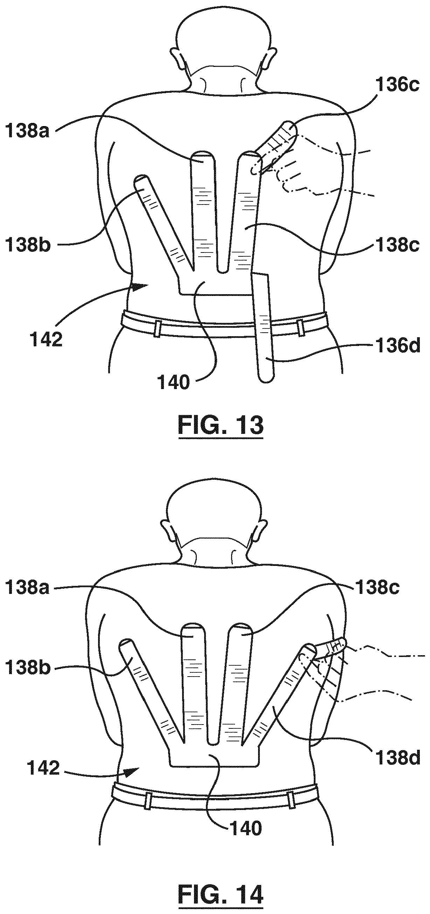

Referring to Figures 13 and 14, the additional finger portions 114b-114d may then be applied to the lower back in a similar fashion to the finger portion 114a. That is, for each additional finger portion 114b-114d, the respective second release liner portions 136b-136d (also referred to herein as additional release liner portions) may be removed from the adhesive side 104 of the additional finger portions 114b-114d of the tape 102, while retaining the finger reinforcement layers 138b-138d (also referred to herein as additional finger reinforcement layers) on the non-adhesive side 106 of the additional finger portions 114b-114d. The adhesive side 106 of the additional finger portions 114b-114d may then be adhered to the lower back, so that the tape 102 lies generally flat on the lower back.

During application of the tape 102, the user may view the instructions, for example the first set of instructions 130, the second set of instructions 132, and/or any separate instructions, and adhere the anchor portion 112 and/or finger portions 114a-114d in an order the accords with the instructions. For example, as the portions of the release liner 108 and reinforcement layer 110 on the anchor portion 112 are marked with the numeral 1, the user may apply the anchor portion 112 first. As the portions of the release liner 108 and reinforcement layer 110 on the finger portion 114a is marked with the numeral 2, the user may apply the finger portion 114a second, and so on.

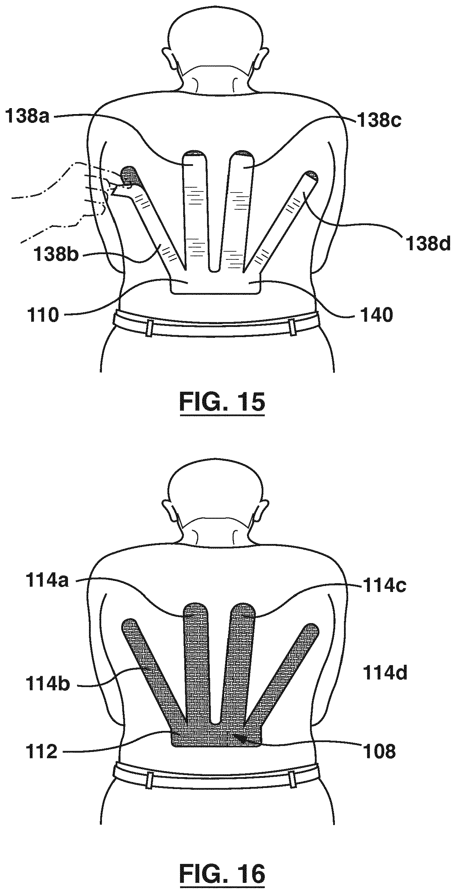

Referring to Figures 15 and 16, the reinforcement layer 110 may then be removed from the tape 102. In the example shown, as the reinforcement layer 110 is a single piece covering substantially all of the tape 102, it may be removed in a single peeling step (i.e. the finger reinforcement layers 138a-d and anchor reinforcement layer 140 may be removed together).

In alternative examples, the reinforcement layer 110 may be weakened along one or more junctions between the anchor portion 112 and the finger portions 114, as shown in Figure 20, or provided as separate pieces on the anchor portion 112 and the finger portions 114, as shown in Figure 21. In such examples, the reinforcement layer 110 may be removed in a stepwise fashion. For example, with reference to Figure 20, the anchor reinforcement layer 2040 may be retained on the anchor portion during application of the anchor portion, and may be used to inhibit stretching of the anchor portion while adhering the adhesive side of the anchor portion to the lower back. Following application of the anchor portion, the reinforcement layer 2010 may be torn along the line 2024, and the anchor reinforcement layer 2040 may be removed from the non-adhesive side of the anchor portion. The finger reinforcement layers 2038a-2038d may be removed in a stepwise fashion, either after each respective finger portion is applied to the lower back, or after all of the finger portions have been applied to the lower back. A similar method may be used to apply the support article 2100 of Figure 21, however the step of tearing the reinforcement layer may be omitted.

In further alternative examples (not shown), as mentioned above, the reinforcement layer 110 may include only finger reinforcement layers 138a-138d, and no anchor reinforcement layer 140. In such examples, the finger reinforcement layers 138a-138d may be removed stepwise, either after each respective finger portion 114a-114d is applied to the lower back 142, or after all of the finger portions 114a-114d have been applied to the lower back 142.

## Claims

1. A support article (100) for the lower back, comprising:
a) a precut piece of kinesiology tape (102) having an adhesive side (104) and a non-adhesive side (106), the precut piece of kinesiology tape comprising an anchor portion (112) for adhering to the lower back, and a plurality of finger portions (114a-114d) extending transversely from a first side (116) of the anchor portion (112) for adhering to the lower back;
b) a release liner (108) on the adhesive side (104) of the precut piece of kinesiology tape (102), the release liner weakened along one or more junctions between the anchor portion (112) and the finger portions (114a-114d); and
c) a stretch-inhibiting reinforcement layer (110) adhered to the non-adhesive side (106) of the precut piece of kinesiology tape (102), the stretch-inhibiting reinforcement layer (110) weakened along the one or more junctions between the anchor portion (112) and the finger portions (114a-114d) or provided as separate pieces on the anchor portion (112) and the finger portions (114a-114d).

2. The support article of claim 1, wherein the finger portions (114a-114d) extend from only the first side (116) of the anchor portion (112).

3. The support article of claim 1 or 2, wherein the release liner (108) has a release liner thickness, and the stretch-inhibiting reinforcement layer (110) has a reinforcement layer thickness less than the release liner thickness.

4. The support article of anyone of claims 1 to 3, further comprising a first set of instructions (130) printed on the stretch inhibiting reinforcement layer (110).

5. The support article of claim 4, further comprising a second set of instructions (132) printed on the release liner (108).

6. The support article of any one of claims 1 to 5, wherein the stretch-inhibiting reinforcement layer (110) is a paper layer.

7. The support article of any one of claims 1 to 6, wherein the release liner (108) is perforated along the one or more junctions.

8. The support article of any one of claims 1 to 7, wherein the release liner (108) includes fold lines thereon for facilitating folding of the release liner during removal.

9. A system for supporting the lower back, comprising:
a) the support article (100) of any of claims 1 to 8, such that the stretch-inhibiting reinforcement layer (110) comprises a plurality of finger reinforcement layers (138a-138d) and an anchor reinforcement layer (140); and
b) instructions directing a user to remove each finger reinforcement layer (138a-138d) from each respective finger portion (114a-114d) after the respective finger portion (114a-114d) has been adhered to the lower back.

## Patentansprüche

1. Stützartikel (100) für den unteren Rücken, der aufweist:
a) ein zugeschnittenes Stück Kinesiologieband (102) mit einer klebenden Seite (104) und einer nichtklebenden Seite (106), wobei das zugeschnittene Stück Kinesiologieband einen Verankerungsteil (112) zum Ankleben am unteren Rücken und mehrere Fingerteile (114a - 114d), die sich von einer ersten Seite (116) des Verankerungsteils (112) quer erstrecken, zum Ankleben am unteren Rücken aufweist;
b) einen Trennbelag (108) auf der klebenden Seite (104) des zugeschnittenen Stücks Kinesiologieband (102), wobei der Trennbelag entlang einer oder mehr Übergangsstellen zwischen dem Verankerungsteil (112) und den Fingerteilen (114a - 114d) geschwächt ist; und
c) eine dehnungshemmende Verstärkungsschicht (110), die an der nichtklebenden Seite (106) des zugeschnittenen Stücks Kinesiologieband (102) anhaftet, wobei die dehnungshemmende Verstärkungsschicht (110) entlang der einen oder den mehr Übergangsstellen zwischen dem Verankerungsteil (112) und den Fingerteilen (114a - 114d) geschwächt ist oder als separate Stücke am Verankerungsteil (112) und den Fingerteilen (114a - 114d) bereitgestellt wird.

2. Stützartikel (100) nach Anspruch 1, wobei die Fingerteile (114a - 114d) sich nur von der ersten Seite (116) des Verankerungsteils (112) erstrecken.

3. Stützartikel (100) nach Anspruch 1 oder 2, wobei der Trennbelag (108) eine Trennbelagdicke hat und die dehnungshemmende Verstärkungsschicht (110) eine Verstärkungsschichtdicke hat, die kleiner als die Trennbelagdicke ist.

4. Stützartikel (100) nach einem der Ansprüche 1 bis 3, der ferner einen ersten Satz Anweisungen (130) aufweist, die auf die dehnungshemmende Verstärkungsschicht (110) aufgedruckt sind.

5. Stützartikel (100) nach Anspruch 4, der ferner einen zweiten Satz Anweisungen (132) aufweist, die auf den Trennbelag (108) aufgedruckt sind.

6. Stützartikel nach einem der Ansprüche 1 bis 5, wobei die dehnungshemmende Verstärkungsschicht (110) eine Papierschicht ist.

7. Stützartikel nach einem der Ansprüche 1 bis 6, wobei der Trennbelag (108) an der einen oder den mehr Übergangsstellen entlang perforiert ist.

8. Stützartikel nach einem der Ansprüche 1 bis 7, wobei der Trennbelag (108) Faltlinien daran zum Ermöglichen des Faltens des Trennbelags während des Entfernens enthält.

9. System zum Stützen des unteren Rückens, das aufweist:
a) den Stützartikel (100) nach einem der Ansprüche 1 bis 8, so dass die dehnungshemmende Verstärkungsschicht (110) mehrere Fingerverstärkungsschichten (138a - 138d) und eine Verankerungsverstärkungsschicht (140) aufweist; und
b) Anweisungen, die einen Benutzer anweisen, jede Fingerverstärkungsschicht (138a - 138d) von jedem jeweiligen Fingerteil (114a - 114d) zu entfernen, nachdem der jeweilige Fingerteil (114a - 114d) am unteren Rücken angeklebt worden ist.

## Revendications

1. Article de soutien (100) pour le bas du dos, comprenant :
a) un morceau précoupé de bande de kinésiologie (102) présentant un côté adhésif (104) et un côté non adhésif (106), le morceau précoupé de bande de kinésiologie comprenant une partie d'ancrage (112) destinée à être collée sur le bas du dos, et une pluralité de parties de pattes (114a-114d) s'étendant transversalement depuis un premier côté (116) de la partie d'ancrage (112) destinées à être collées sur le bas du dos ;
b) une protection anti-adhésive (108) sur le côté adhésif (104) du morceau précoupé de bande de kinésiologie (102), la protection anti-adhésive étant affaiblie le long d'une ou de plusieurs jonctions entre la partie d'ancrage (112) et les parties de pattes (114a-114d) ; et
c) une couche de renforcement anti-étirement (110) collée sur le côté non adhésif (106) du morceau précoupé de bande de kinésiologie (102), la couche de renforcement anti-étirement (110) étant affaiblie le long d'une ou de plusieurs jonctions entre la partie d'ancrage (112) et les parties de pattes (114a-114d) ou étant fournie en tant que morceaux séparés sur la partie d'ancrage (112) et les parties de pattes (114a-114d).

2. Article de soutien selon la revendication 1, dans lequel les parties de pattes (114a-114d) s'étendent uniquement depuis le premier côté (116) de la partie d'ancrage (112).

3. Article de soutien selon la revendication 1 ou 2, dans lequel la protection anti-adhésive (108) a une épaisseur de protection anti-adhésive, et la couche de renforcement anti-étirement (110) a une épaisseur de couche de renforcement inférieure à l'épaisseur de protection anti-adhésive.

4. Article de soutien selon l'une quelconque des revendications 1 à 3, comprenant en outre un premier ensemble d'instructions (130) imprimé sur la couche de renforcement anti-étirement (110).

5. Article de soutien selon la revendication 4, comprenant en outre un second ensemble d'instructions (132) imprimé sur la protection anti-adhésive (108).

6. Article de soutien selon l'une quelconque des revendications 1 à 5, dans lequel la couche de renforcement anti-étirement (110) est une couche de papier.

7. Article de soutien selon l'une quelconque des revendications 1 à 6, dans lequel la couche de protection anti-adhésive (108) est perforée le long d'une ou de plusieurs jonctions.

8. Article de soutien selon l'une quelconque des revendications 1 à 7, dans lequel la couche de protection anti-adhésive (108) comporte des lignes de pliage pour faciliter le pliage de la protection anti-adhésive lors de son retrait.

9. Système de soutien pour le bas du dos, comprenant :
a) l'article de soutien (100) selon l'une quelconque des revendications 1 à 8, de telle sorte que la couche de renforcement anti-étirement (110) comprenne une pluralité de couches de renforcement de pattes (138a-138d) et une couche de renforcement d'ancrage (140) ; et
b) des instructions instruisant un utilisateur d'ôter chaque couche de renforcement de patte (128a-138d) de chaque partie de patte respective (114a-114d) après que la partie de patte respective (114a-114d) a été collée sur le bas du dos.
